# EUROPEAN PATENT SPECIFICATION

(11) **EP 2 935 164 B1**
(45) Date of publication and mention of the grant of the patent: **19.09.2018**
(21) Application number: 13801591.2
(22) Date of filing: 09.12.2013
(51) Int. Cl.: C07C 17/02, C07C 17/156, C07C 17/25, C07C 19/045, C07C 21/06

(54) **PROCESS FOR THE MANUFACTURE OF ETHYLENE DICHLORIDE (EDC)**
VERFAHREN ZUR HERSTELLUNG VON ETHYLENDICHLORID (EDC)
PROCÉDÉ DE FABRICATION DE DICHLORURE D'ÉTHYLÈNE (EDC)

(30) Priority: 20.12.2012 EP 12198593
(43) Date of publication of application: 28.10.2015
(73) Proprietor: Solvay SA, 1120 Bruxelles (BE)
(72) Inventor: DEGRAEVE, Paul Julius, B-7822 Isières (BE); MARTIN CARNICERO, Maria, B-1060 Brussels (BE)
(74) Representative: King, Alex
(86) International application number: PCT/EP2013/075884
(87) International publication number: WO 2014/095441

(56) References cited:
- WO-A1-01/21564
- WO-A1-2010/034392
- WO-A1-2012/110371
- US-B1- 7 182 840

## Description

This application claims priority to European application No. 12198593.1 filed December 20, 2012.

The present invention relates to a process for the manufacture of ethylene dichloride (EDC).

For producing vinyl chloride monomer (VCM), two methods are generally employed : the hydrochlorination of acetylene and the dehydrochlorination of ethylene dichloride (1,2-dichloroethane) or EDC. The latter generally happens by thermal cracking and the EDC used therefore is generally obtained by direct chlorination and/or oxychlorination of ethylene.

As namely explained in "Chemical Process Design : Computer-Aided Case Studies", Alexandre C. Dimian and Costin Sorin Bildea, Copyright© 2008 WILEY-VCH Verlag GmbH & Co. KGaA, Weinheim, ISBN : 978-3-527-31403-4, Chapter 7 entitled : "Vinyl Chloride Monomer Process", to date, most of the VCM technologies are based on "balanced" processes.
By this is meant that all intermediates and by-products are recycled in a way that ensures a tight closure of the material balance to only VCM as the final product, starting from ethylene, chlorine and oxygen. The main chemical steps involved are :
1. Direct chlorination of ethylene to 1,2 - ethylene dichloride (EDC) :

   C2H4+Cl2→C2H4Cl2+218kJ/mol
2. Thermal cracking (pyrolysis) of EDC to VCM :

   C2H4Cl2→C2H3Cl+HCl-71kJ/mol
3. Recovery of HCl and oxychlorination of ethylene to EDC :

   C2H4+2HCl+0.502→C2H4Cl2+H2O+238kJ/mol

Hence, an ideal balanced process can be described by the overall equation :

C2H4+0.5Cl2+0.25O2→C2H3Cl+0.5H2O+192.5kJ/mol.

This document gives, namely in sub-chapter 7.6, a flow chart (Figure 7.8) showing how the EDC (both "fresh" EDC coming from the (oxy)chlorination and recycled EDC, which has not been cracked and has been separated from the pyrolysis reaction products (VCM and the HCl)) is purified prior to being fed to the pyrolysis reactor. Such a purification is generally carried out in at least 3 steps : first, a purification in "light" impurities (having a boiling point below the one of EDC), generally using a distillation column ; then, a purification in "heavy" impurities (having a boiling point above the one of EDC), generally also using a distillation column (heavy ends column) and finally, on the bottom products of the latter, which still contains EDC in order to allow advanced removal of impurities, a concentration step, also using a distillation column (heavy ends concentration column).

This same document sets forth, namely in sub-chapter 7.7, several ways of saving energy in a "balanced" process as described above. However, none of them involves energy savings on the heavy ends column.

US 4,788,357 discloses a process involving an energy saving on this very column by adiabatically condensing the top product and by using the heat of condensation so obtained to heat up the reboiler at the bottom of the column.

US 7,182,840 also discloses a process involving an energy saving on this very column by heating up the boiler thereof, but it does so by recovering the reaction heat of the direct chlorination of ethylene into EDC.

WO 2001/21564 describes a process for the production of 1,2-dichloroethane from chlorine and ethylene by direct chlorination wherein the heat developed in the direct chlorination reactor is recovered.

WO 2010/034392 relates to a method for using the reaction heat developing during the production process of 1,2-dichloroethane from ethylene in a fluidized bed reactor wherein said reaction heat is dissipated through cooling pipe bundles located inside the reactor and positioned in the fluidized bed.

WO 2012/110371 describes a process for the manufacture of ethylene dichloride according to which the ethylene-containing composition is subjected to chlorination and/or oxychlorination in order to produce ethylene dichloride, further pyrolyzed in order to produce vinyl chloride, further polymerized in order to produce polyvinyl chloride. In one embodiment, this process uses a succession of heat exchanges optimizing the use of energy.

The present invention aims at providing a new route for energy saving in an EDC manufacturing process. It is based on the idea of heating up the feed of the heavy ends column leading to a reduction of thermal duty of its reboiler, and doing so also by using heat available on the EDC manufacturing process itself. To this end, the invention relates to a process for the manufacture of EDC according to claim 1.

In the above, the terms « substantially pure» or "substantially free from" mean in fact that there only remains a limited amount of impurities (typically : a few w% or less) in said streams.

The terms "EDC stream" intend to designate a stream of fluid, gaseous or liquid, but generally liquid, comprising preferably more than 90 % and more preferably more than 95 % and even more preferably more than 97 % of EDC, the remainder being lower or higher boiling impurities i.e. compounds having a boiling point lower or a higher than the one of EDC. Typical impurities are VCM, ethylchloride, 11EDC, chloroprene (α and β), carbon trichloride, carbon tetrachloride, trichlorethane, perchlorethylene, tetrachlorethane, dichlorobutane, dichlorobutene, tars.

Lower and higher boiling impurities, generally called light ends and heavy ends, are preferably both separated in order to obtain a purified EDC that can be used to feed the furnaces of the cracking section of a VCM plant or that can be sold on the market. Hence, in a preferred embodiment of the invention, light ends are separated in a first step, preferably in a distillation column. This column can also be used simultaneously as dewatering column. Depending on the plant, this column can be dedicated to one source of EDC (in this case several light ends columns are generally installed) or common for different EDC sources.

The distillation column(s) used in the process of the invention is a fractionating column, device widely used in the chemical process industries where a multi-component mixture must be separated in groups of compounds within a relatively small range of boiling points, also called fractions. The "lightest" products with the lowest boiling points exit from the top of the column and the "heaviest" products with the highest boiling points exit from the bottom.

Inside the column, the down flowing reflux liquid provides cooling and condensation of up flowing vapours thereby increasing the efficacy of the distillation column. The more reflux and/or more trays provided, the better is the separation of lower boiling materials from higher boiling materials.

Bubble-cap "trays" or or sieve "trays" or valve "trays" are one of the types of physical devices which can be used to provide good contact between the up flowing vapour and the down flowing liquid inside an industrial fractionating column.

However, in the frame of the invention, it might be advantageous to use a packing material instead of trays, because it allows a lower pressure drop across the column. This packing material can either be random dumped packing such as Pall, CMR or Raschig rings (1-3" wide) or structured sheet metal.

According to the invention, the EDC stream fed to the heavy ends distillation column is at least partly heated up by using waste heat available in the EDC manufacturing process. Preferably, the complete feed is heated up.

According to the invention, a shell and tube type or a falling film type heat exchanger can be used to heat up the EDC stream fed to the heavy ends column.

Tube and shell heat exchangers can be provided in either a vertical position with the heating fluid in the shell side or in the horizontal position with the heating fluid in the tubes.

Preferably, the heater is a falling film heat exchanger. Falling film exchanger are generally heat exchangers with vertical tubes, where a fluid is heated and partially evaporated while flowing as a thin film downward on the heated tube-walls. Falling film heat exchangers are preferred because they allow a low difference between the temperature of the heating medium on shell side and the temperature of the fluid heated and evaporating inside the tubes.

In the invention, the waste heat used in the heater is generated by compressing part of the top stream of substantially pure EDC from the heavy ends column itself using a Vapour Recompression (VR) device. If vapour compression is performed by a mechanically-driven compressor or blower, this evaporation process is usually referred to as MVR (Mechanical Vapour Recompression). In case of compression performed by high pressure motive vapour ejectors, the process is usually called TVR (Thermal Vapour Recompression) or thermocompression or Vapour Compression. The latter (TVR) allows saving less thermal energy but it does not require any additional mechanical power consumption.

The EDC obtained by a process as described above can be used in a process for the manufacture of vinyl chloride monomer (VCM) by pyrolysis of a purified EDC.

The conditions under which the pyrolysis may be carried out are known to persons skilled in the art. This pyrolysis is advantageously obtained by a reaction in the gaseous phase in a tubular oven. The usual pyrolysis temperatures are between 400 and 600°C with a preference for the range between 480°C and 540°C. The residence time is advantageously between 1 and 60 s with a preference for the range from 5 to 25 s. The rate of conversion of the EDC is advantageously limited to 45 to 75 % in order to limit the formation of by-products and the fouling of the tubes of the oven.

The VCM obtained as described above can be used in a process for the manufacture of PVC.

The process for the manufacture of PVC may be a mass, solution or aqueous dispersion polymerization process ; preferably, it is an aqueous dispersion polymerization process.

The expression "aqueous dispersion polymerization" is understood to mean free radical polymerization in aqueous suspension as well as free radical polymerization in aqueous emulsion and polymerization in aqueous microsuspension.

The expression "free radical polymerization in aqueous suspension" is understood to mean any free radical polymerization process performed in aqueous medium in the presence of dispersing agents and oil-soluble free radical initiators.

The expression "free radical polymerization in aqueous emulsion" is understood to mean any free radical polymerization process performed in aqueous medium in the presence of emulsifying agents and water-soluble free radical initiators.

The expression "aqueous microsuspension polymerization", also called polymerization in homogenized aqueous dispersion, is understood to mean any free radical polymerization process in which oil-soluble initiators are used and an emulsion of droplets of monomers is prepared by virtue of a powerful mechanical stirring and the presence of emulsifying agents.

The present invention is illustrated in a non limitative way by figures 1 to 9 attached, of which Figures 3 and 4 show some preferred embodiments thereof by comparison with prior art. In these figures, identical reference numbers designate identical or similar items.

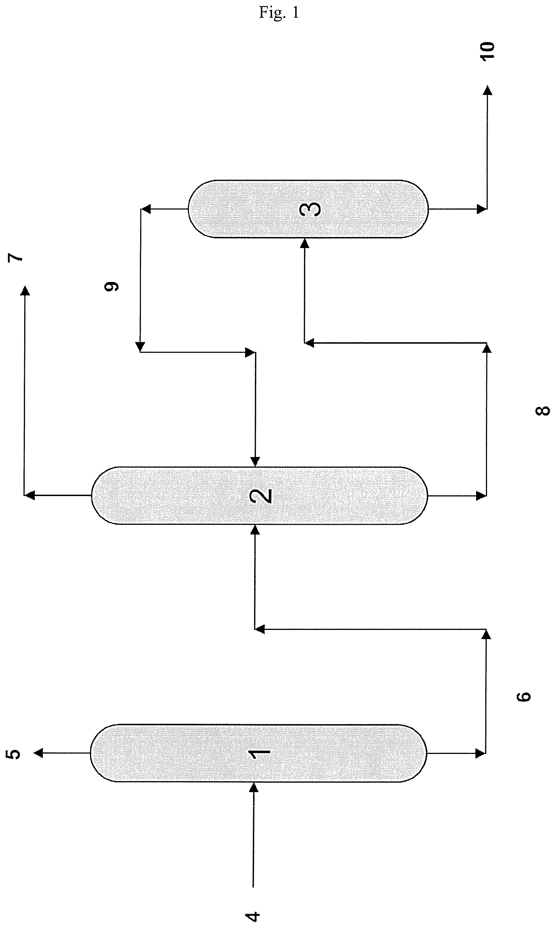

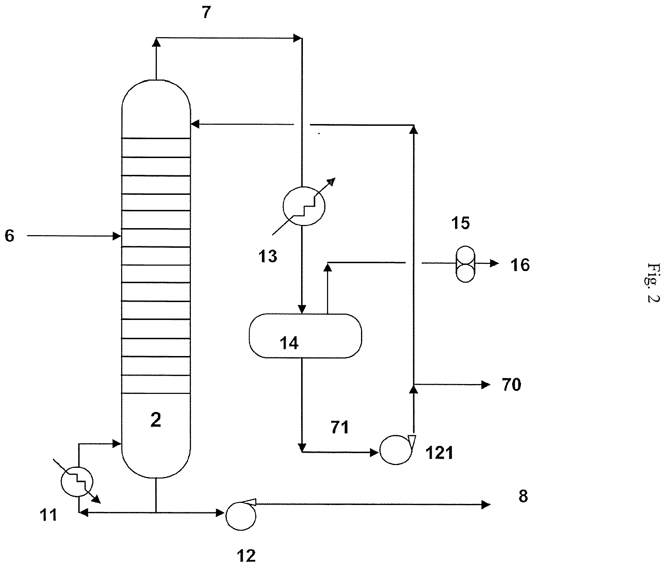

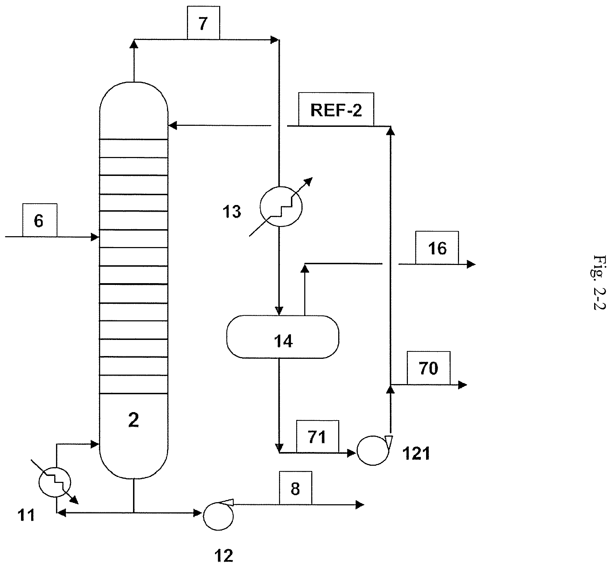

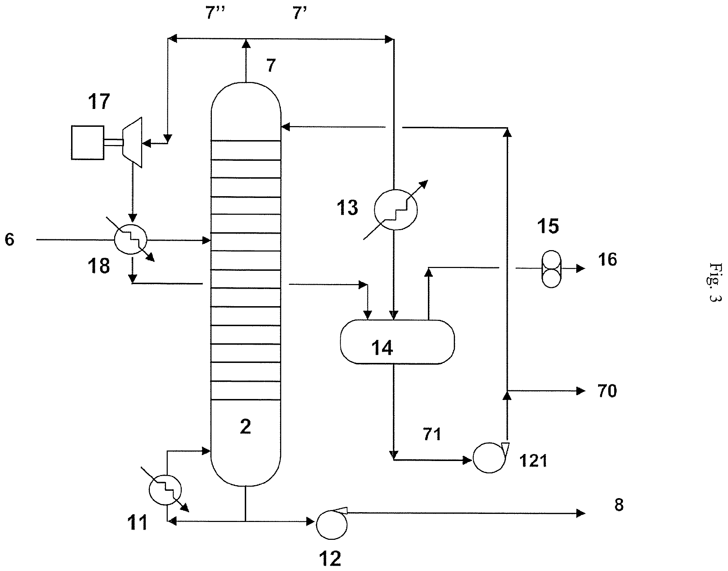

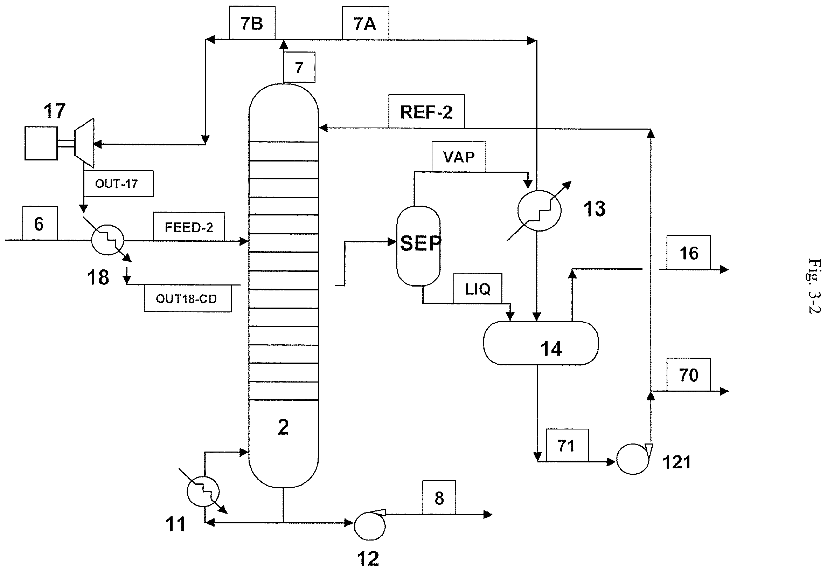

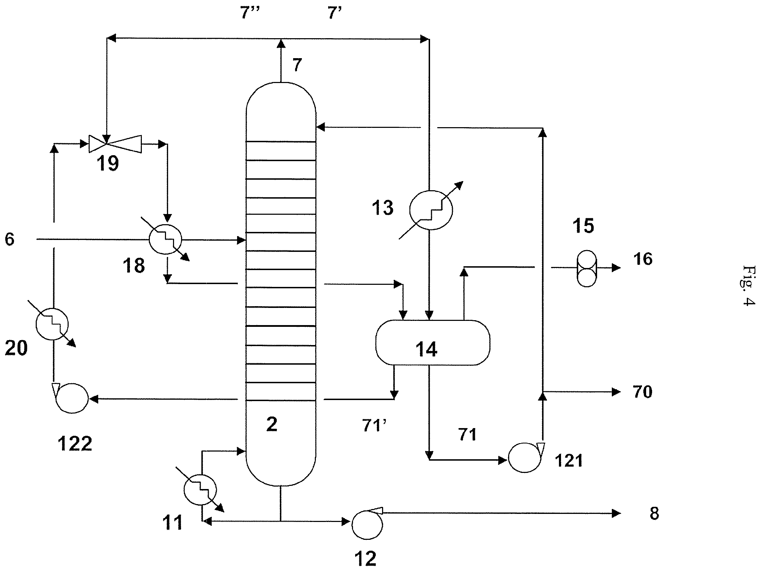

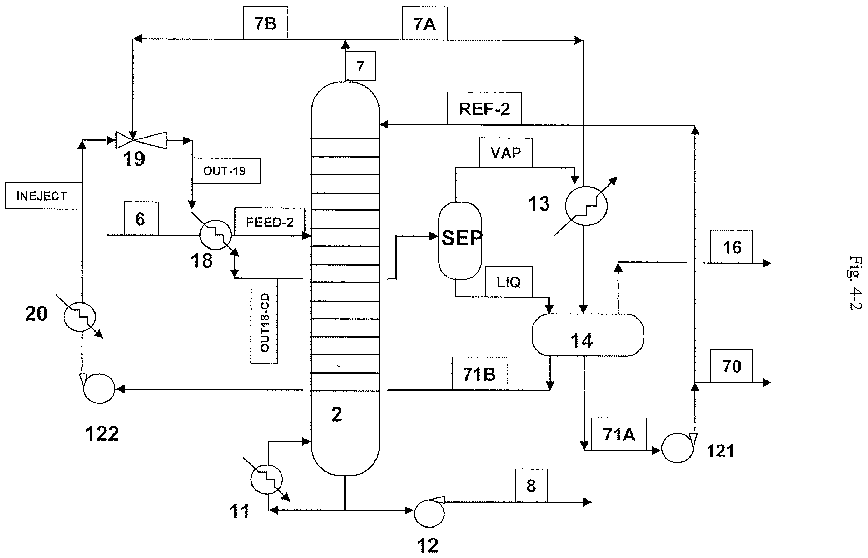

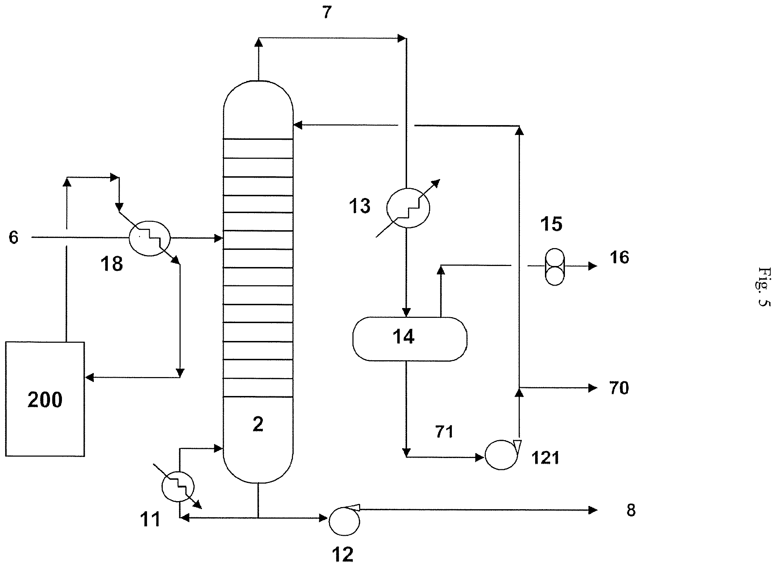

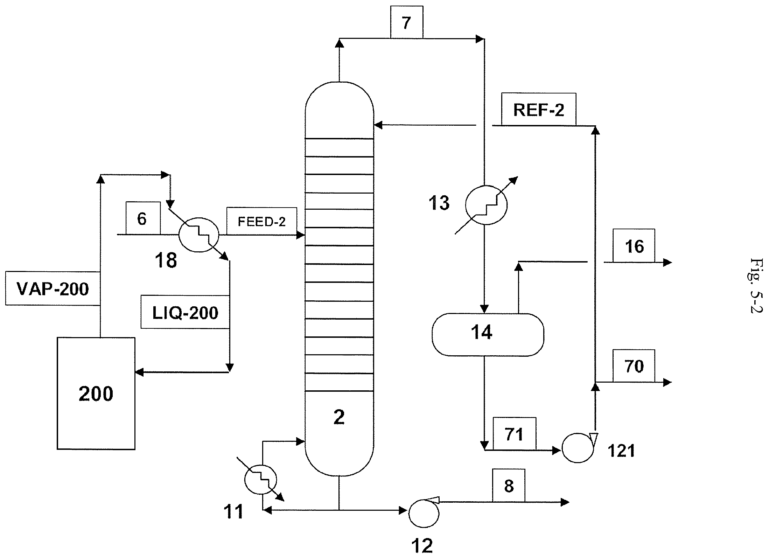

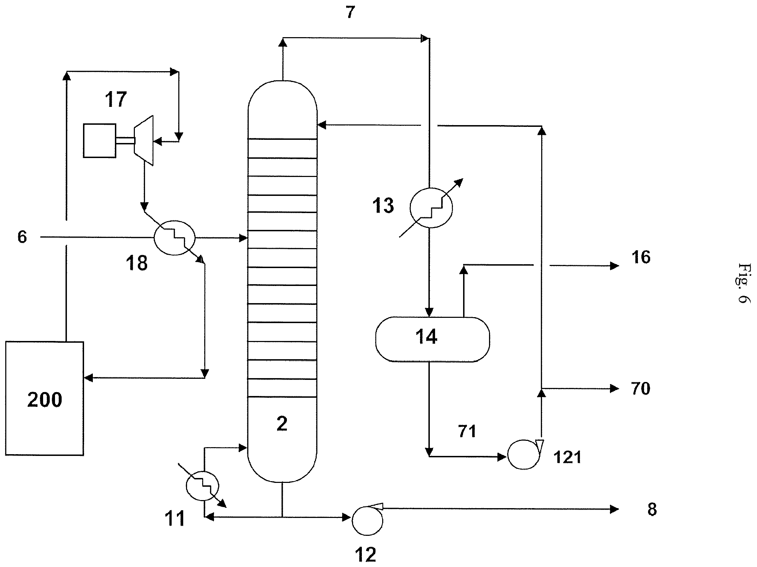

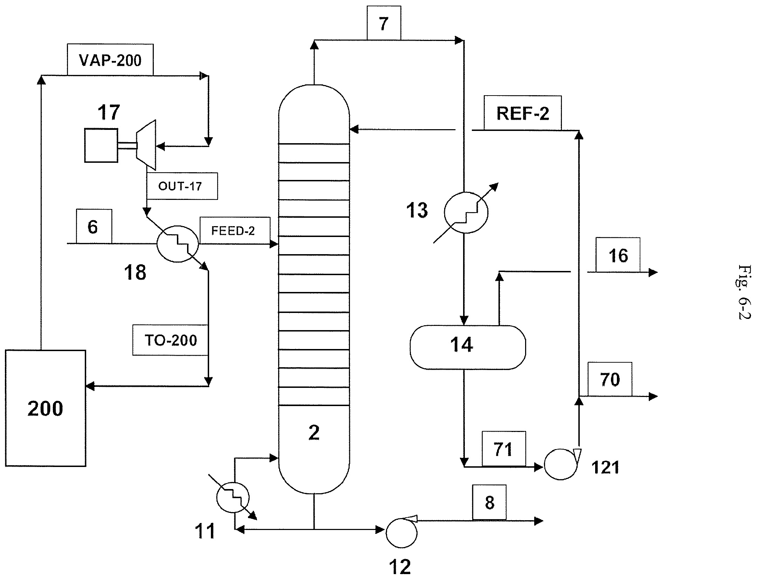

Figures 1 and 2 show typical arrangements for EDC purification columns, figures 3 and 4 show different embodiments of arrangements according to the invention and Figures 5 to 9 show alternative arrangements not according to the invention. More precisely :
- Figure 1 shows the disposal of typical EDC purification columns
- Figure 2 details a heavy ends column and the devices typically associated therewith
- Figure 3 shows an embodiment of the invention in which the waste heat is coming from a MVR device compressing part of the top stream of substantially pure EDC from the heavy ends column
- Figure 4 shows an embodiment of the invention in which the waste heat is coming from a TVR device compressing part of the top stream of substantially pure EDC from the heavy ends column
- Figure 5 shows an alternative to the invention in which the
   waste heat is directly and completely coming from a direct chlorination unit of ethylene to
   EDC
- Figure 6 shows an alternative to the invention in which the waste heat is coming from the top of a direct chlorination unit of ethylene to EDC that has additionally been compressed using a MVR

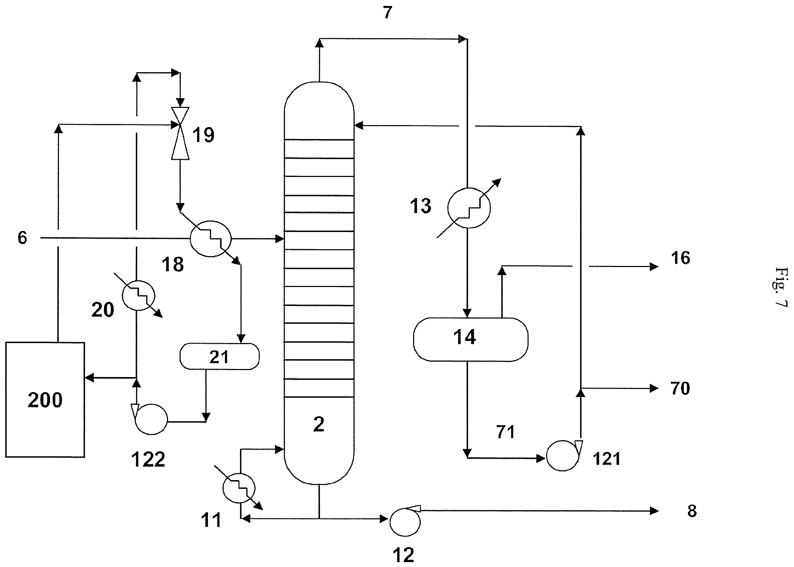

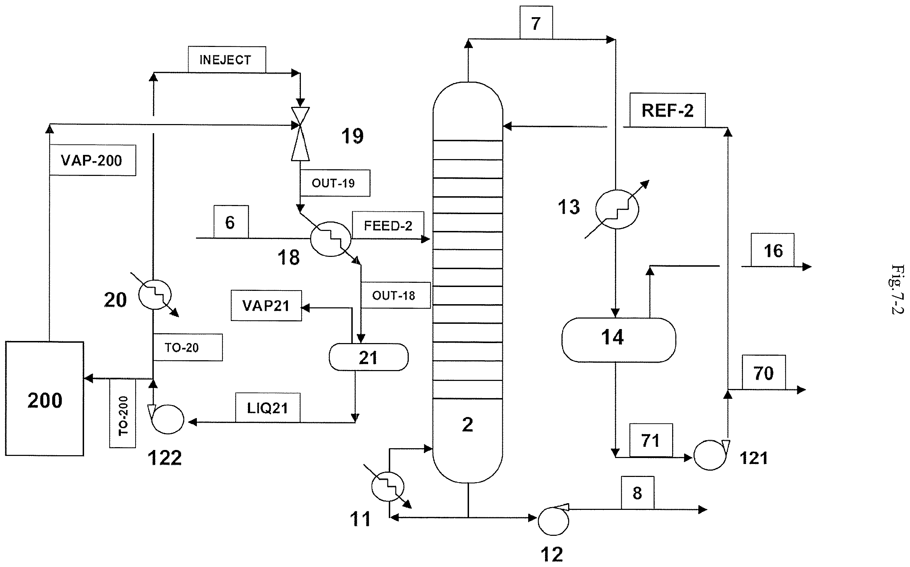

- Figure 7 shows an alternative to the invention in which the waste heat is coming from the top of a direct chlorination unit of ethylene to EDC that has additionally been compressed using a TVR

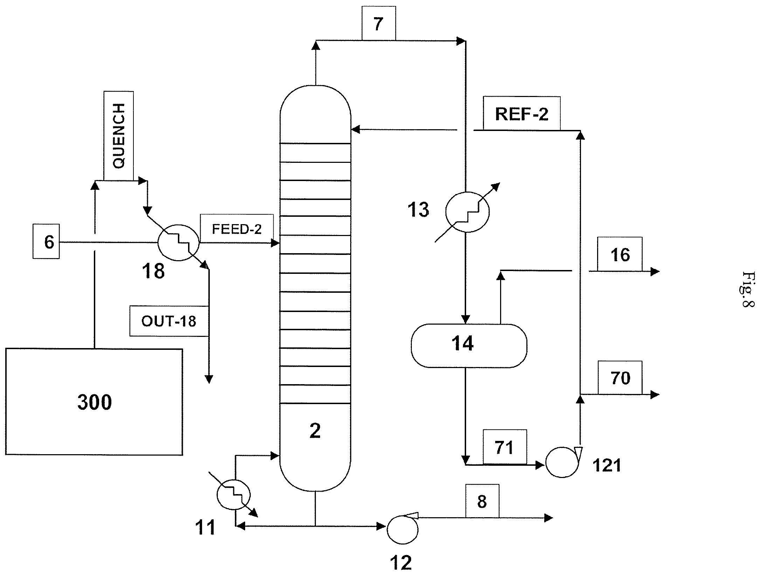

- Figure 8 shows an alternative to the invention in which the waste heat is coming from the quenching device of an oxychlorination unit of ethylene to EDC

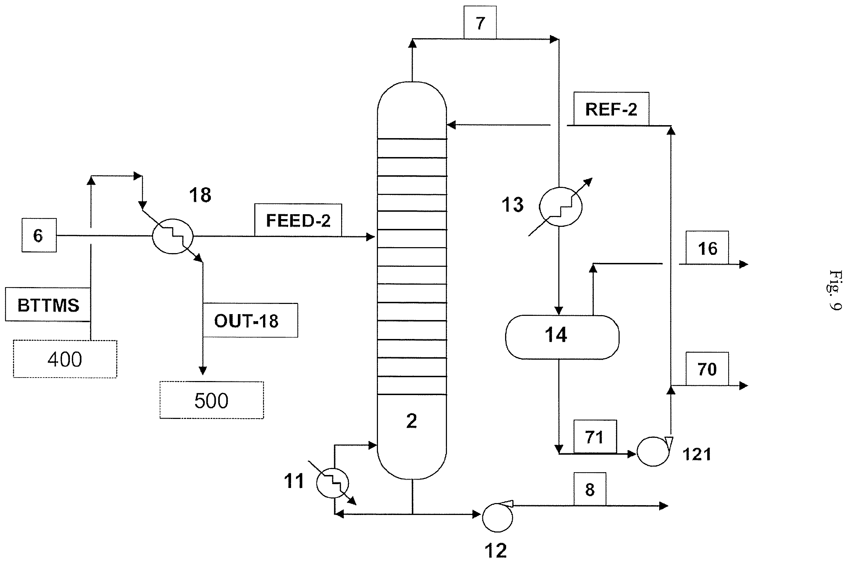

- Figure 9 shows an alternative to the invention in which the waste heat is coming from the bottom of the VCM purification column.

In these figures, similar devices have similar or identical reference numbers.

As can be seen from figure 1, in a typical EDC purification process, a feed of impure EDC (4) is separated in a first distillation column (1) (or light ends column) in light ends at the top (5) and in a stream (6) of EDC containing heavy impurities at the bottom. This stream is fed to a second distillation column (2) (or heavy ends column), where it is separated again in a top stream of substantially pure EDC (7) and a bottom stream of EDC enriched in heavy ends (8). This bottom stream is sent to a concentration column (3) where it is separated in a stream of substantially pure EDC (9) which is sent back to column (2), and to heavy ends (10) which can for instance be further treated or eliminated (for instance by incineration).

In this typical arrangement, all 3 purification column are classical distillation columns with reboiler, condenser...

Figure 2 details the heavy ends column (2) and associated devices, wherein the top stream of substantially pure EDC (7) leaving said column (2) is first condensed in a condenser (13) and then, sent to a reflux drum (14) from which vent gases (16) are separated, eventually using a vacuum system (15), from a liquid stream of purified EDC (71) which is partly recycled as reflux to column (2) using a pump (121). The other part of purified EDC is send to a downstream application (70), like a pyrolysis step to VCM. A reboiler (11) is used to heat the bottom of column (2) and the bottom stream of EDC enriched in heavy ends (8) is removed from the column (2) using a pump (12).

In a first embodiment of the invention, illustrated in Figure 3, one part (7') of stream (7) is treated as explained above (condensed in a condenser (13) and sent to a reflux drum (14)), while the rest (7") of said stream (7) is sent to a MVR (17) which generates heat used in a heat exchanger (18) heating up the feed (6) of the column (2).

Figure 4 shows a second embodiment of the invention, identical to the one of Figure 3 except for the MVR (17) which is replaced by a TVR (19) also receiving vapour from an evaporator (20) fed with an EDC stream (71') leaving the reflux drum (14) and conveyed to said evaporator (20) through a pump (122).

A first alternative to the invention is shown in Figure 5, which has a layout quite similar to the one of Figure 4 but where a hot stream coming from a direct chlorination reactor (200) is used in the heat exchanger (18) heating up the feed (6) of column (2).

Figures 6 and 7 show two other alternatives to the invention quite similar to the one of Figure 5 but where a VR is inserted between the direct chlorination reactor (200) and the heat exchanger (18).

This VR is either a MVR (17) as shown in Figure 6, or a TVR (19) as shown in Figure 7. In the latter case, the TVR also receives high pressure vapour coming from an evaporator (20) which evaporates part of the stream condensed in exchanger (18), collected in drum (21) and pumped through pump (122), the other part of said stream being returned to the direct chlorination reactor (200).

Figure 8 shows a fourth alternative to the invention in which the waste heat used to heat up the feed (6) of column (2) is a hot stream leaving the quenching device (300) of an oxychlorination unit of ethylene to EDC.

Figure 9 shows a fifth alternative to the invention in which the waste heat used to heat up the feed (6) of column (2) is a hot stream leaving the bottom of the VCM purification column (400) before it is sent to the light end removal step (500) of a VCM production process as described above.

Table 1 hereafter shows the results of simulation/calculations made using the 7.2 release of the AspenONE® software.

These results are based on the layouts of Figures 2-2 to 7-2 (which are slight modified versions of corresponding Figures 2 to 7 described above) and of Figures 8 and 9 attached, using the working conditions set forth in Tables 2 to 9 (Table 2 giving the working conditions of Figure 2-2 etc... until Table 9, which gives those of Figure 9).

The layout of the Figures used for the calculations incorporates the flows from Tables 2 to 9, which are all put into squares (to avoid confusion with device features).

The energy savings indicated in Table 1 (as negative figures, the positive ones concerning new energy consumptions) for the layouts of Figures 3-2 to 9 are all calculated versus the energy consumption of the layout of Figure 2-2.

The embodiment of Figure 3 and the alternative of Figure 6 (and Figures 3-2 and 6-2) require additional electricity consumption, while the embodiment of Figure 4 and the alternative of Figure 7 (and 4-2 and 7-2) require additional medium pressure steam consumption in the evaporator (20). However, they all lead to net energy savings.

The embodiments of figures 3 and 4 allow recovery of waste heat available on top of the heavy end column. These embodiments avoid any connexion to other parts of EDC or VCM production. They also allow a reduction of cooling capacity on top of the heavy end column. The sub-embodiment with the MVR allows the highest heat recovery but requires new electricity consumption.

The alternatives of figures 5, 6 and 7 allow recovery of waste heat available in the production of EDC by direct chlorination. Depending on the level of temperature of the gaseous EDC flow available in the chlorination section, waste heat can be used directly or a VR device is required. Installation of a VR device is more expensive. The MVR solution requires additional electricity. These embodiments lead to a reduction of cooling capacity in chlorination section, while cooling capacity on top of heavy end column increases slightly.

The alternative of figure 8 allows recovering waste heat available in oxychlorination and leads to a reduction of cooling capacity in the oxychlorination section, while cooling capacity on top of the heavy end column increases slightly.

The alternative of figure 9 allows recovering waste heat available in the hot EDC stream leaving the bottom of the VCM column.

**Table 1**

| Concerned item or property | Item number | Units | Fig 2-2 | Fig 3-2 | Fig 4-2 | Fig 5-2 | Fig 6-2 | Fig 7-2 | Fig.8 | Fig.9 |
|---|---|---|---|---|---|---|---|---|---|---|
| boiler | 11 | kW | 9001 | 6565 | 6565 | 6565 | 6565 | 6565 | 6565 | 8435 |
| condenser | 13 | kW | 10616 | 7879 | 9477 | 11106 | 11106 | 11106 | 11106 | 10720 |
| T out 13 | - | °C | 35 | 35 | 35 | 35 | 35 | 35 | 35 | 35 |
| T reflux column 2 | - | °C | 35,1 | 56,9 | 54,3 | 35,1 | 35,1 | 35,1 | 35,1 | 35,1 |
| Reflux rate column2 | - | t/h | 28,5 | 35,6 | 35,2 | 33,0 | 33,0 | 33,0 | 33,0 | 29,4 |
| compressor | 17 | kW | - | 244 | - | - | 143 | - | - | - |
| preheater column 2 | 18 | kW | - | 2927 | 2927 | 2927 | 2927 | 2927 | 2927 | 670 |
| preh.ejector | 20 | kW | - | - | 1772 | - | - | 1394 | - | - |
| Motion flow rate (71B/TO-20) | - | t/h | - | - | 15 | - | - | 14 | - | - |
| Motion flow ratio to ejector | 19 | Mass/Mass | - | - | 0,56 | - | - | 0,63 | - | - |
| Boiler savings | 11 | % | - | -27,1% | -27,1% | -27,1% | -27,1% | -27,1% | -27,1% | -6,3% |
| CD variation | 13 | % | - | -25,8% | -10,7% | 4,6% | 4,6% | 4,6% | 4,6% | 1,0% |
| MPS savings in Boiler alone | 11 | kW | - | -2436 | -2436 | -2436 | -2436 | -2436 | -2436 | -566 |
| Net MPS savings | 11-20 | kW | - | -2436 | -664 | -2436 | -2436 | -1042 | -2436 | -566 |
| New electricity consumption | 17 | kW | - | 244 | - | - | 143 | - | - | - |
| CD variation | 13 | kW | - | -2737 | -1139 | 490 | 490 | 490 | 490 | 104 |

| | | | | | | | | | | |
|---|---|---|---|---|---|---|---|---|---|---|
| MPS= Medium Pressure Steam CD= condenser | | | | | | | | | | |

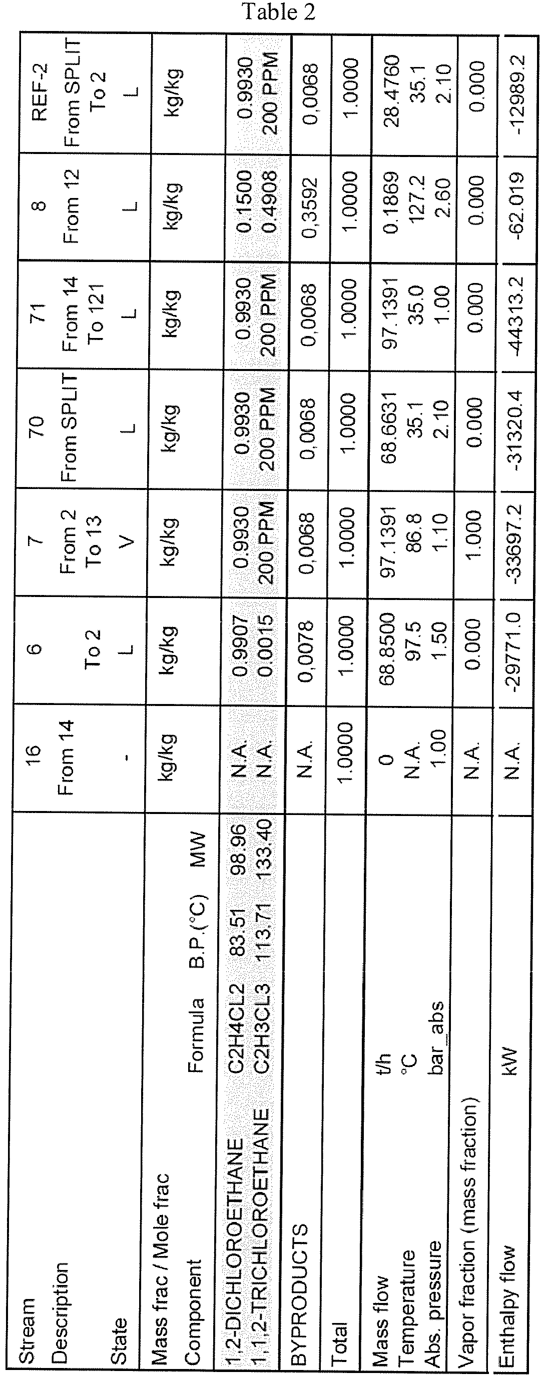

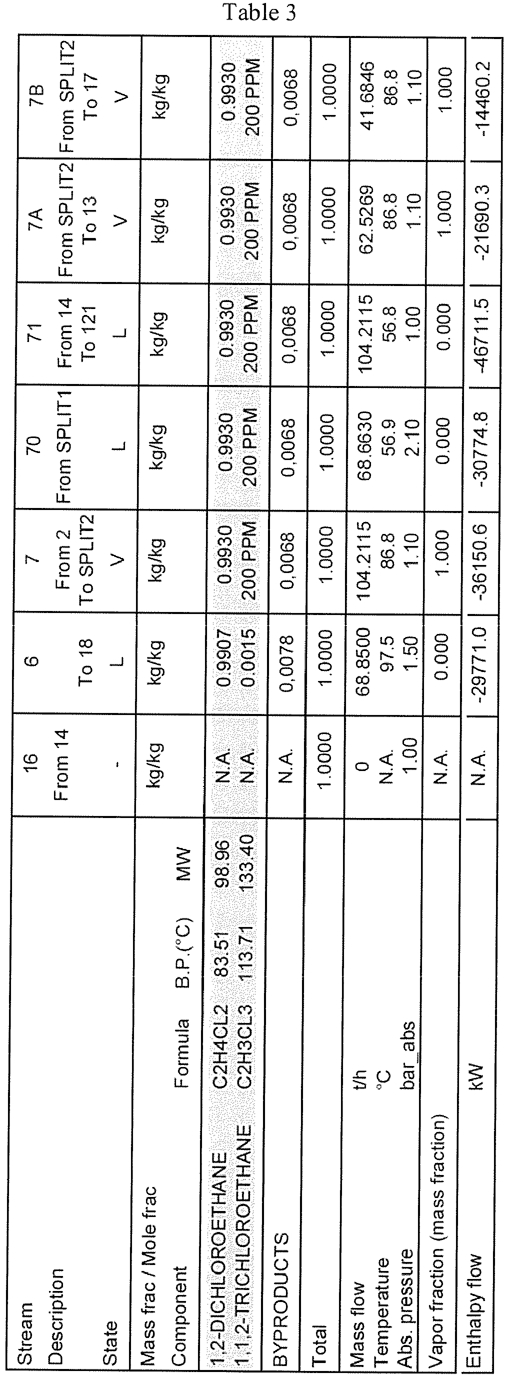

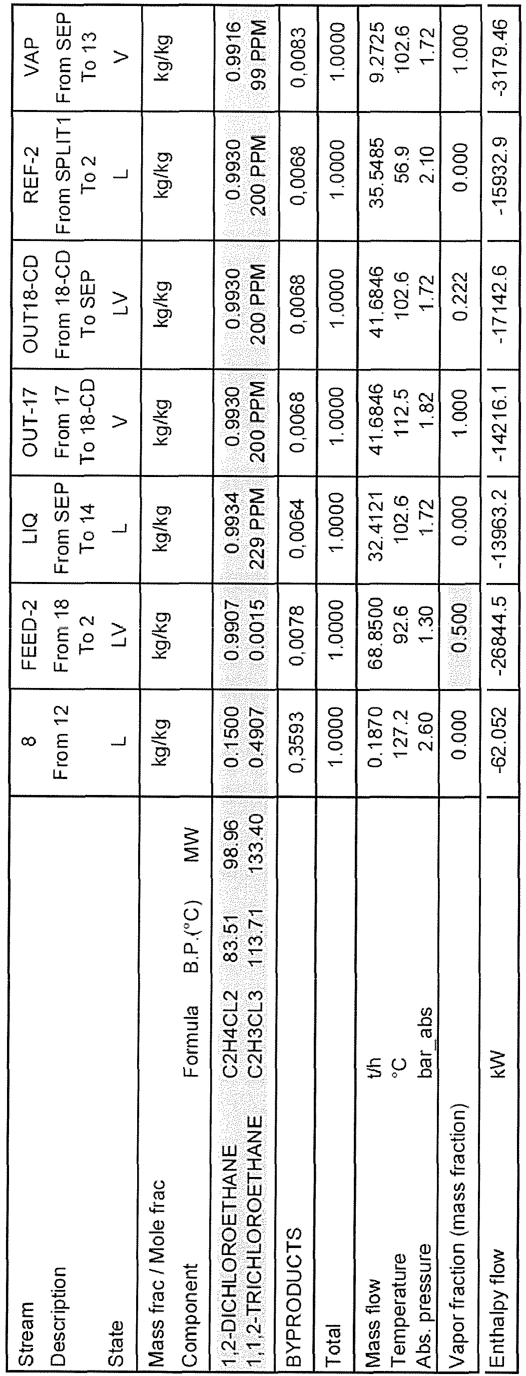

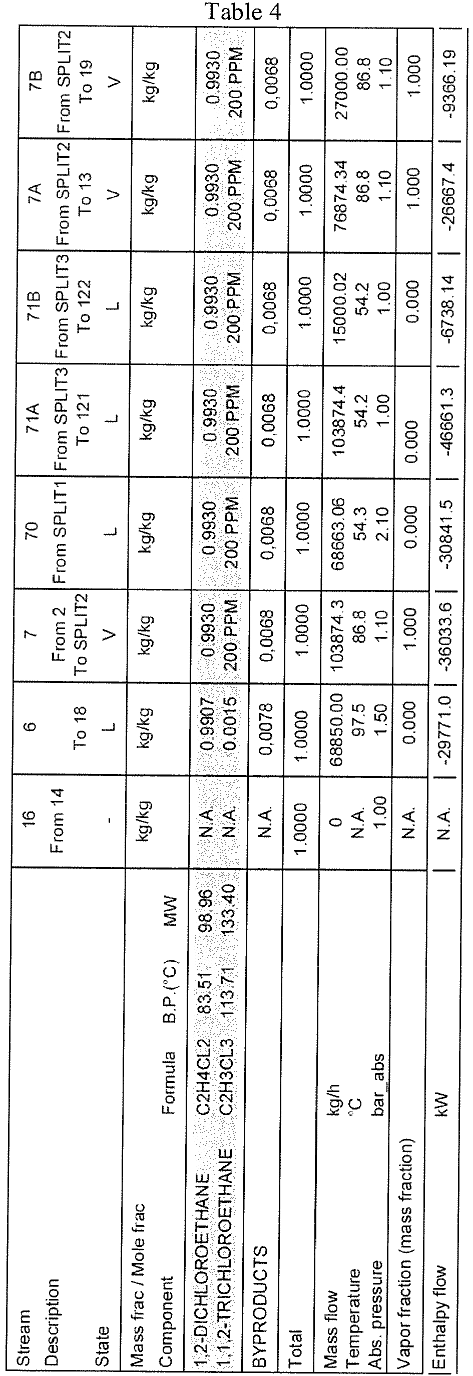

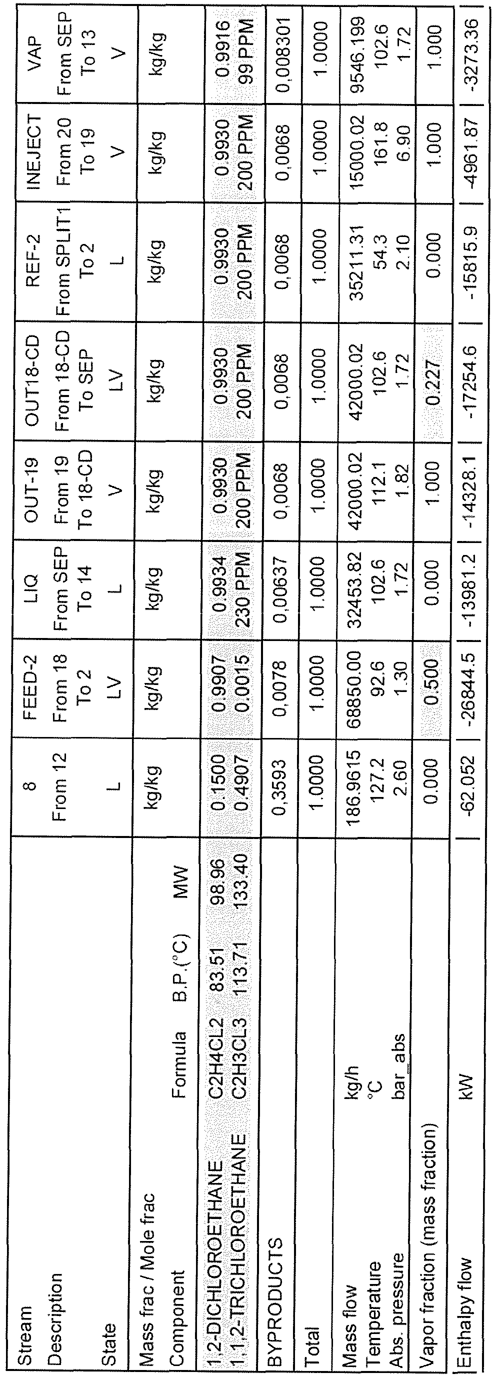

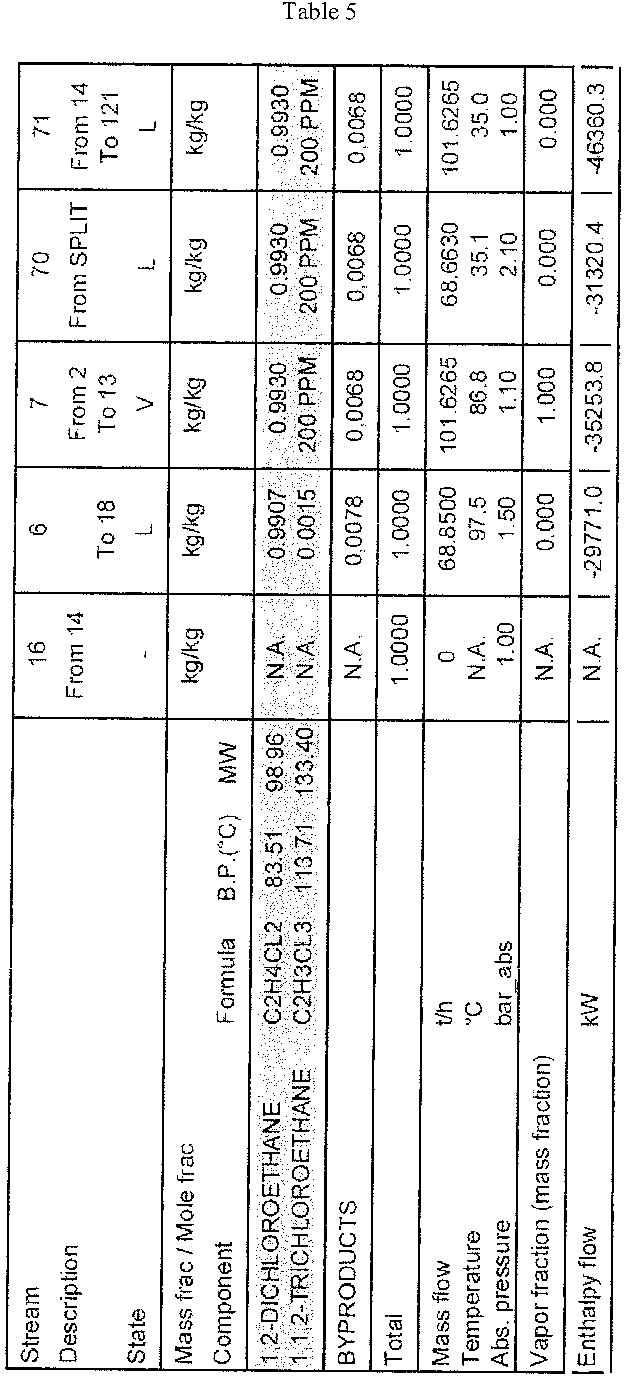

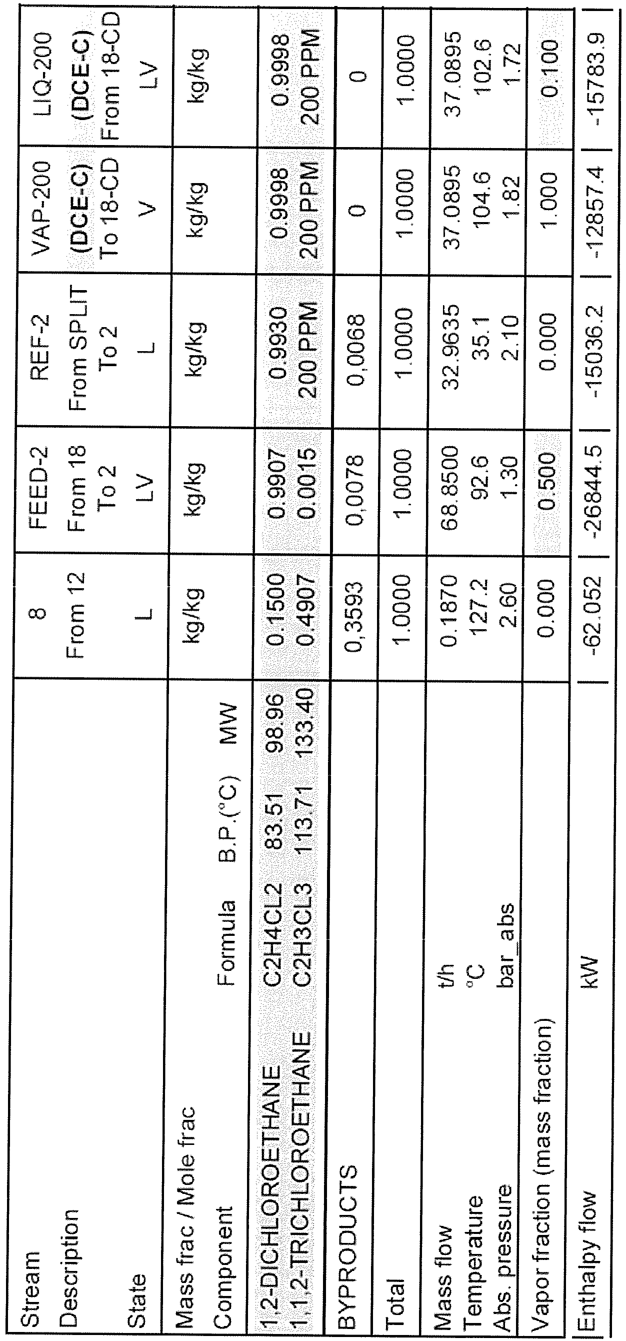

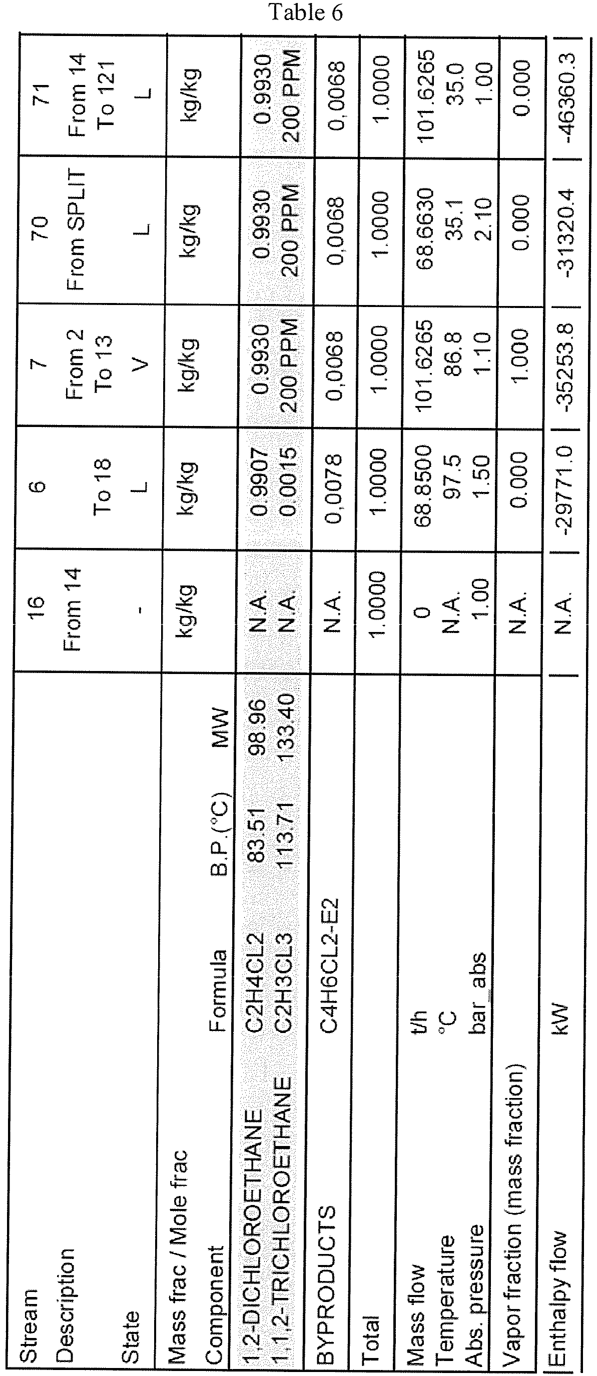

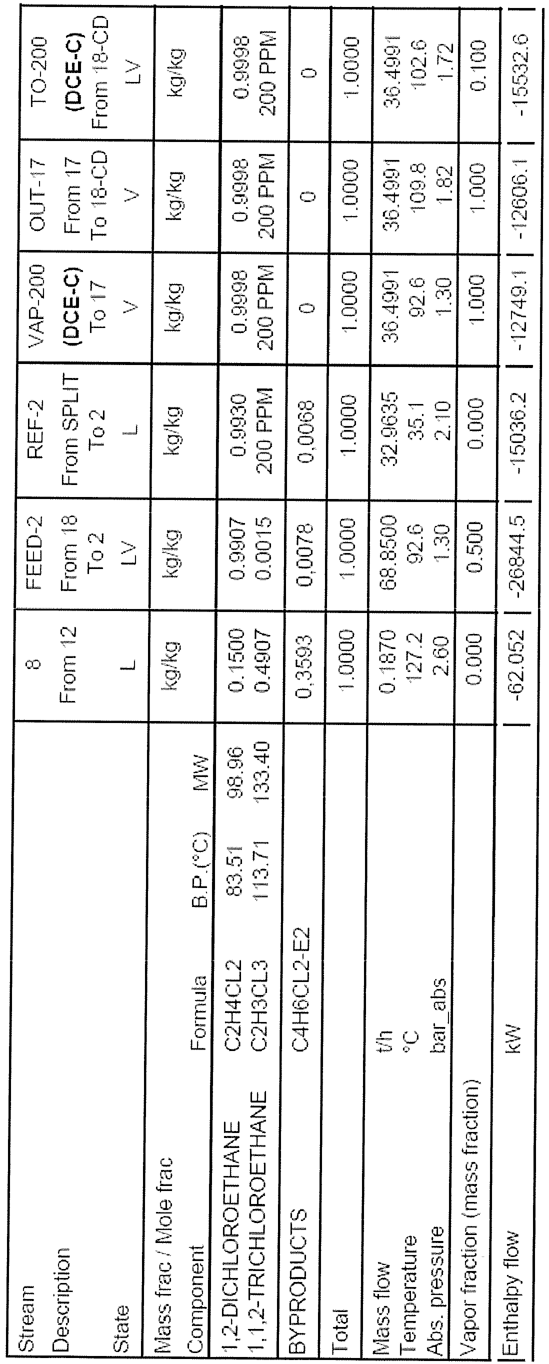

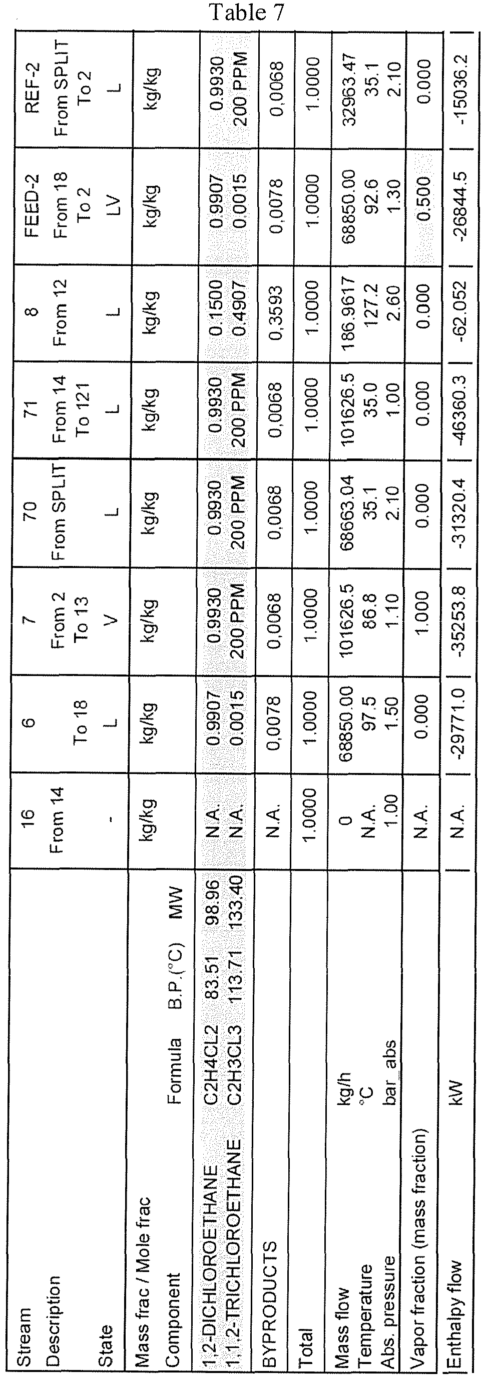

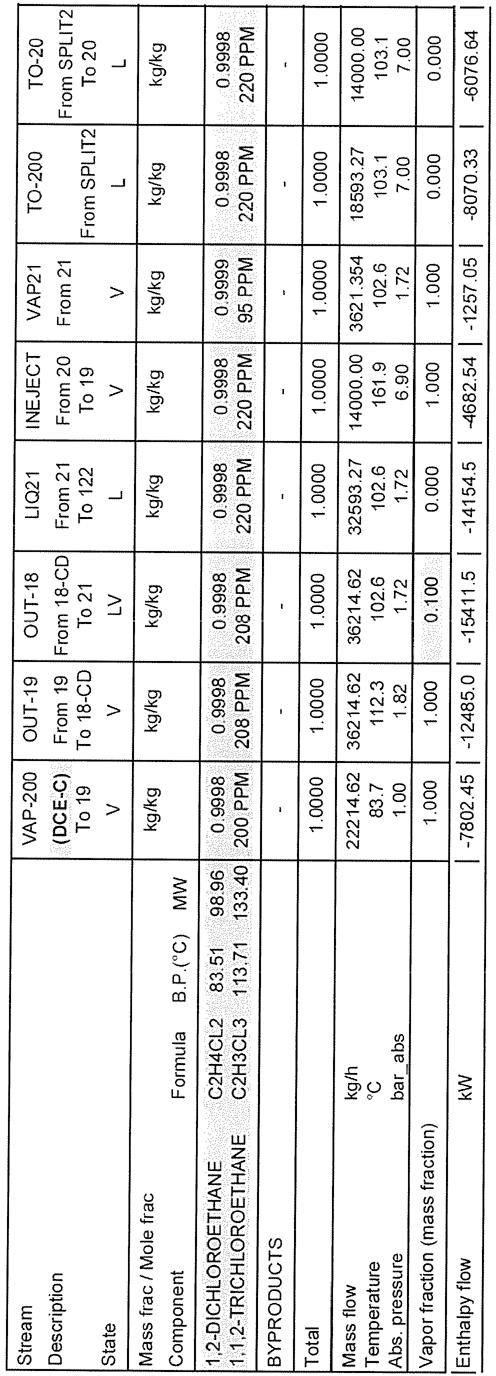

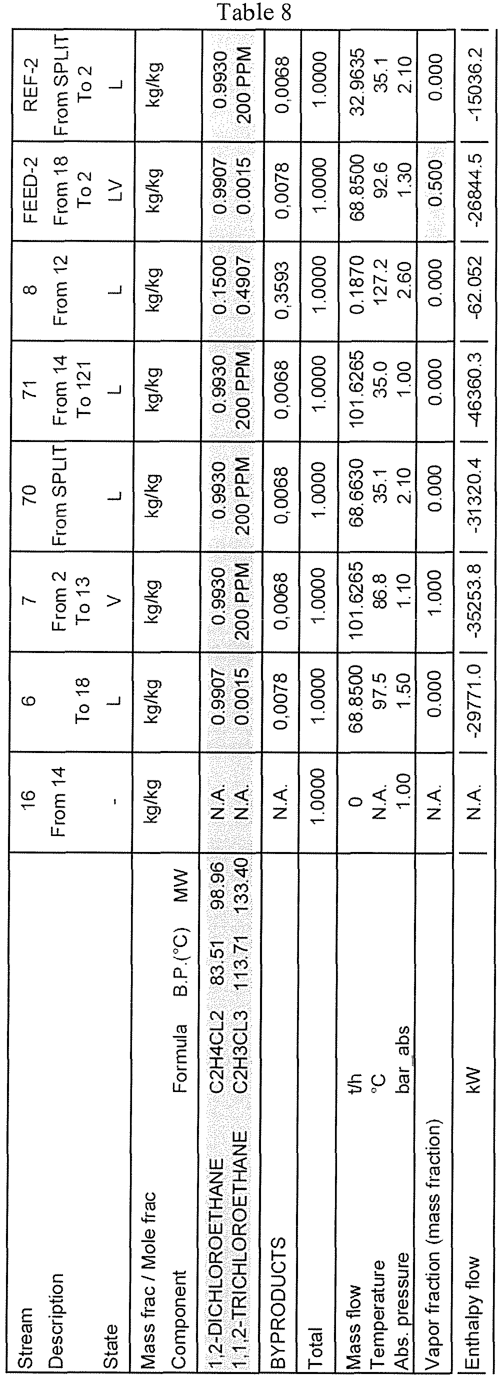

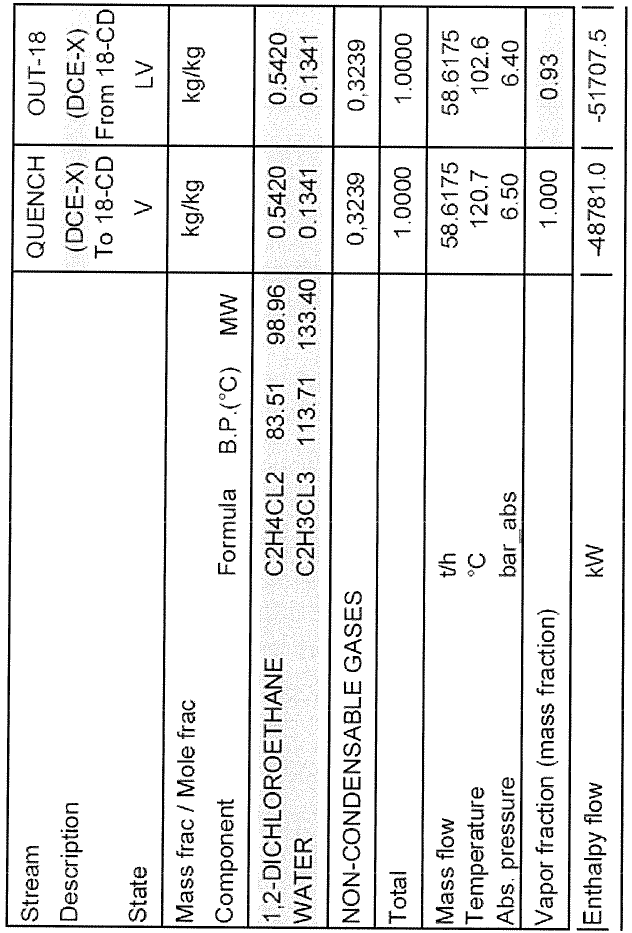

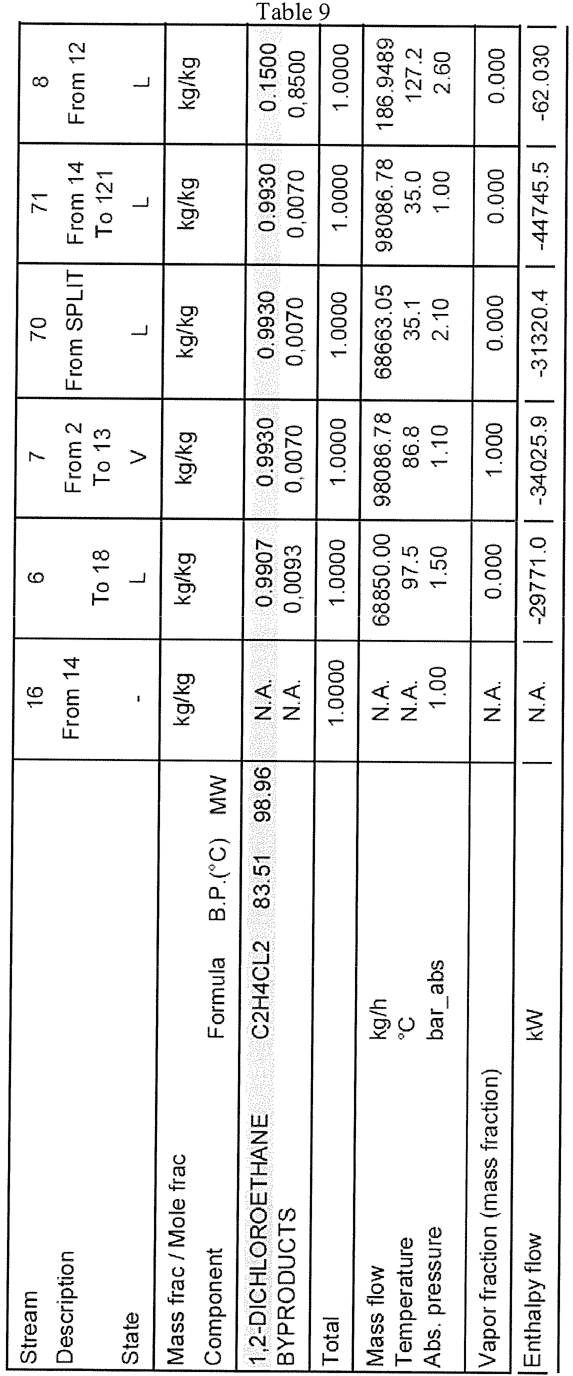

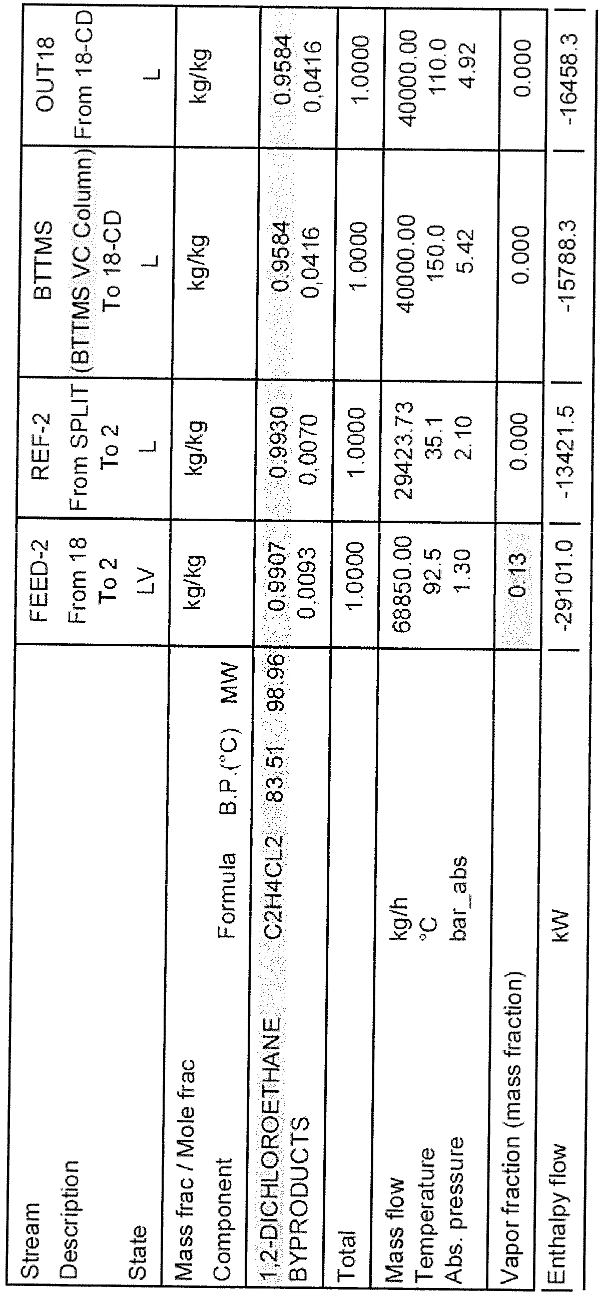

## Claims

1. Process for the manufacture of EDC or Ethylene Di-Chloride by chlorination and/or oxychlorination of ethylene, said process comprising the purification of an EDC stream according to the following steps :
- eventually removing the lower boiling impurities from the EDC stream so as to generate a stream of EDC substantially free from lower boiling impurities ;
- feeding this EDC stream to a heavy ends distillation column so as to get a top stream of substantially pure EDC and a bottom stream comprising higher boiling impurities and EDC,
wherein the EDC stream fed to the heavy ends distillation column is at least partly heated up by a heater using waste heat available in the EDC manufacturing process itself or in a subsequent pyrolysis step of said EDC into vinyl chloride monomer or VCM, and wherein the waste heat used in the heater is generated by compressing part of the top stream of substantially pure EDC from the heavy ends column using a Vapour Recompression or VR device.

2. Process according to claim 1, wherein the heavy ends distillation column is a fractionating column comprising packing material.

3. Process according to any of the preceding claims, wherein the heavy ends distillation column operates under vacuum.

4. Process according to any of the preceding claims, wherein the complete EDC stream fed to the heavy ends distillation column is heated up by the heater.

5. Process according to any of the preceding claims, wherein the heater is a falling film type heat exchanger or a shell and tube type heat exchanger.

6. Process according to any of the preceding claims, wherein the VR device is a MVR or Mechanical Vapour Recompression device, or a TVR or Thermal Vapour Recompression device.

## Patentansprüche

1. Verfahren zur Herstellung von EDC oder Ethylendichlorid durch Chlorierung und/oder Oxychlorierung von Ethylen, wobei das Verfahren die Reinigung eines EDC-Stroms gemäß den folgenden Schritten umfasst:
- schließlich Entfernen der niedriger siedenden Verunreinigungen aus dem EDC-Strom, wobei ein EDC-Strom entsteht, der im Wesentlichen frei von niedriger siedenden Verunreinigungen ist;
- Zuführen dieses EDC-Stroms zu einer Nachlaufdestillationskolonne, wobei man einen Kopfstrom aus im Wesentlichen reinem EDC und einen Sumpfstrom, der höhersiedende Verunreinigungen und EDC umfasst, erhält;
wobei der der Nachlaufdestillationskolonne zugeführte EDC-Strom durch einen Heizer unter Nutzung von Abwärme, die im EDC-Produktionsvorgang selbst oder in einem anschließenden Schritt der Pyrolyse des EDC zu Vinylchlorid-Monomer oder VCM verfügbar ist, wenigstens zum Teil aufgeheizt wird und wobei die in dem Heizer genutzte Abwärme dadurch erzeugt wird, dass man einen Teil des Kopfstroms aus im Wesentlichen reinem EDC aus der Nachlaufkolonne unter Verwendung einer Dampfrekompressions- oder VR-Vorrichtung verdichtet.

2. Verfahren gemäß Anspruch 1, wobei die Nachlaufdestillationskolonne eine Fraktionierungskolonne ist, die Füllkörper umfasst.

3. Verfahren gemäß einem der vorstehenden Ansprüche, wobei die Nachlaufdestillationskolonne unter Vakuum arbeitet.

4. Verfahren gemäß einem der vorstehenden Ansprüche, wobei der vollständige EDC-Strom, der der Nachlaufdestillationskolonne zugeführt wird, durch den Heizer aufgeheizt wird.

5. Verfahren gemäß einem der vorstehenden Ansprüche, wobei der Heizer ein Wärmetauscher des Fallfilmtyps oder ein Wärmetauscher des Rohrbündeltyps ist.

6. Verfahren gemäß einem der vorstehenden Ansprüche, wobei die VR-Vorrichtung eine MVR oder mechanische Dampfrekompressionsvorrichtung oder eine TVR oder thermische Dampfrekompressionsvorrichtung ist.

## Revendications

1. Procédé pour la fabrication d'EDC ou Di-Chlorure d'Ethylène par chloration et/ou oxychloration d'éthylène, ledit procédé comprenant la purification d'un courant d'EDC selon les étapes suivantes :
- éventuellement élimination des impuretés de faible point d'ébullition du courant d'EDC afin de produire un courant d'EDC pratiquement exempt d'impuretés de faible point d'ébullition ;
- introduction de ce courant d'EDC dans une colonne de distillation de fractions lourdes afin d'obtenir un courant de tête d'EDC pratiquement pur et un courant de queue comprenant des impuretés de point d'ébullition élevé et EDC,
dans lequel le courant d'EDC introduit dans la colonne de distillation de fractions lourdes est au moins partiellement réchauffé par un dispositif de chauffage utilisant de la chaleur perdue disponible dans le procédé de fabrication d'EDC lui-même ou dans une étape de pyrolyse subséquente dudit EDC en monomère de chlorure de vinyle ou VCM, et dans lequel la chaleur perdue utilisée dans le dispositif de chauffage est générée en comprimant une partie du courant de tête d'EDC pratiquement pur à partir de la colonne de fractions lourdes en utilisant une Recompression de Vapeur ou un dispositif VR.

2. Procédé selon la revendication 1, dans lequel la colonne de distillation de fractions lourdes est une colonne de fractionnement comprenant un matériau de garnissage.

3. Procédé selon l'une quelconque des revendications précédentes, dans laquelle la colonne de distillation de fractions lourdes fonctionne sous vide.

4. Procédé selon l'une quelconque des revendications précédentes, dans lequel le courant d'EDC complet introduit dans la colonne de distillation de fractions lourdes est réchauffé par le dispositif de chauffage.

5. Procédé selon l'une quelconque des revendications précédentes, dans lequel le dispositif de chauffage est un échangeur de chaleur de type à film ruisselant ou un échangeur de chaleur de type tube et calandre.

6. Procédé selon l'une quelconque des revendications précédentes, dans lequel le dispositif VR est un MVR ou Dispositif de Recompression de Vapeur Mécanique, ou un TVR ou Dispositif de Recompression de Vapeur Thermique.
